Europäisches Patentamt

European Patent Office

Office européen des brevets

(19)

(11) Publication number: **0 038 150**
A1

# (12) EUROPEAN PATENT APPLICATION

(21) Application number: **81301451.1**

(22) Date of filing: **02.04.81**

(51) Int. Cl.³: **G 01 N 33/54**

(30) Priority: **10.04.80 JP 46256/80**

(43) Date of publication of application: **21.10.81**
**Bulletin 81/42**

(84) Designated Contracting States: **AT BE CH DE FR GB IT LI LU NL SE**

(71) Applicant: **FUJIZOKI PHARMACEUTICAL CO.LTD., 6-7, Shimoochiai 4-chome Shinjuku-ku, Tokyo 161 (JP)**

(72) Inventor: **Kasahara, Yasushi, 310, Estate Nagayama 3-4-4 Nagayama 3-chome, Tama-shi Tokyo (JP)**
Inventor: **Tsuji, Yoshikatsu, 607, Kitamikata Takatsu-ku, Kawasaki-shi, Kanagawa-ken (JP)**

(74) Representative: **Silverman, Warren et al, HASELTINE LAKE & CO. Hazlitt House 28 Southampton Buildings Chancery Lane, London WC2A 1AT (GB)**

(54) **Sero-diagnostic method for syphilis and other diseases.**

(57)  A diagnostic method for syphilis and other diseases capable of serodiagnosis is disclosed in which a test sample is caused to react with an antigen bonded support material which is used as a solid phase, the antigen being derived from the pathogen in question, and a labelled anti specific antibody is caused to react with the solid phase, whereby the amount of the anti specific antibody may be measured. Alternatively, when two different antibodies are produced in blood at different stages of infection, a plurality of test samples are caused to react with a plurality of such solid phases at the same time, and the solid phases are equally divided into two groups. A labelled anti first specific antibody is caused to react with each of the solid phases in one group, and a labelled second specific antibody is caused to react with each of the solid phases in the other group. Hence, the amount of the anti first specific antibody and the amount of the total anti organism antibody are measured at the same time and enable an indication of the stage of development of the disease to be obtained.

ACTORUM AG

- 1 -

"Sero-diagnostic Method for Syphilis and other Diseases"

This invention relates to a diagnostic method for diseases capable of serodiagnosis, in particular, but not exclusively syphilis, and especially diseases such as syphilis at the initial stages of infection.

The conventional diagnostic methods for syphilis may be summarised as follows. Serodiagnosis is carried out in which Treponema pallidum (hereinafter referred to as "TP"), which is the pathogenic organism of syphilis, is employed as the antigen. Recently, a very simple method utilising hemagglutination has been developed as a serodiagnosis of syphilis. This method is now in general use as a diagnostic method with high specificity and is almost free from non-specific reaction (i.e. false positive reaction). However, when carrying out the conventional diagnostic method employing hemagglutination, although it can be determined whether or not a person is infected with syphilis, it gives no information as to the stage of infection of syphilis, that is, the timing of the infection and the progress thereof. Recently it has been appreciated that even when a person is infected with syphilis in fact, that patient may not develop symptoms of actual syphilis or develops symptoms of syphilis latens because of the effects of medicines, such as antibiotics with which the patient is being dosed for purposes other than the treatment of syphilis.

Therefore, in order to treat syphilis most effectively it is extremely important from a clinical point of view to check accurately the infection of syphilis at the initial stages.

It is therefore an object of the present invention to provide a diagnostic method for diseases, such as syphilis, capable of serodiagnosis which can reliably ensure diagnosis of the disease at an initial stage of infection.

According to one aspect of the present invention there is provided a serodiagnostic method for a disease capable of serodiagnosis, in which method antigen derived from the pathogenic organism of the disease is bonded to a support material to yield organism antigen bonded support material as a solid phase, a test serum sample is caused to react with said solid phase in a first reaction, a labelled anti specific antibody is caused to react with said solid phase in a second reaction to yield a reaction product labelled in an amount corresponding to the amount of anti organism specific antibody present, and the amount of anti organism specific antibody present is measured.

This invention also provides a sero-diagnostic method for a disease capable of serodiagnosis and in which two different antibodies are produced in the blood at different stages of infection, in which method antigen derived from the pathogenic organism of the disease, is bonded to a plurality of support materials to yield organism antigen bonded support materials as solid phases, a plurality of test serum samples is caused to react simultaneously with said solid phases in a first reaction, said solid phases are equally divided into two groups, a labelled anti first specific antibody is caused to react with each of said solid phases in one group and a labelled anti second specific antibody is caused to react with each of said solid phases in the other group in a second reaction to yield reaction products labelled in amounts corresponding to the amounts of anti organism first specific antibody or total anti organism antibody and the respective amounts of anti organism first specific antibody and total anti organism antibody in the sample are measured thereby to indicate through the ratio between said amounts, the stage of infection with the disease.

Although the method of this invention will be described hereinafter with reference to serodiagnosis of syphilis, it is pointed out that it is not limited to serodiagnosis of this disease, but is applicable to all diseases capable of serodiagnosis.

In the immune response with respect to syphilis at the initial stages of its development, a first antibody, IgM (Immunoglobulin M) is first produced in the blood, and another antibody IgG (Immunoglobulin G), is then produced, the amount of the IgG antibody increasing with time. Therefore, to achieve diagnosis of syphilis at the initial infection stages, quantitative measurement of the specific IgM antibody for the antigen, which is the infection source of syphilis, is required. Moreover, by measuring the total amount of the specific antibody at the same time and calculating the ratio of the amount of the specific IgM antibody to that of the total specific antibody, the progress of the syphilis infection can be determined.

In the diagnostic method employing TP, which is now in general use, TP itself is caused to react with a test sample, and then with a fluorescent labelled anti IgM specific antibody, so that the degree of fluorescent dyeing of TP can be determined by visual inspection. This method is called "Fluorescent treponemal antibody test".

In another method of this kind for detecting syphilis in the initial infection stages, an anti IgM specific antibody is fixed to a support material and is then caused to react with a test sample. A labelled TP

0038150

antigen is then applied to the test sample and the amount of the anti TP IgM antibody is then measured.

The former method, however, has shortcomings in that there is a problem in handling since TP itself is employed. Moreover, the anti IgM specific antibody cannot be measured quantitatively. The latter method has different shortcomings in that other IgM antibodies which will have increased in the blood due to infection complications and other forms of IgM which usually exist abundantly in the blood react with the anti TP IgM antibody, thereby affecting the sensitivity and accuracy of the measurement.

The present invention eliminates these conventional shortcomings and provides a diagnostic method for e.g. syphilis with high specificity, capable of enabling the quantities of the anti TP IgM antibody present and the total anti TP antibody to be measured simultaneously with high sensitivity and accuracy.

When carrying out the method of the present invention, a variety of materials, such as beads, test tubes, trays for hemagglutination tests, latexes and Sepharose (Registered Trade Mark of Pharmacia Co.Ltd.) may be employed as support materials. The TP antibody is bonded to the desired support material and the TP antibody bonded support is employed as a solid phase. The solid phase and a test sample are allowed to interact with each other to achieve a first reaction whereby the TP antibody of each globulin class contained in the test sample becomes bonded to the solid phase. The solid phase is then washed with water. In the second reaction, anti

IgM specific (IgM μ-chain) antibody is labelled with, for example, enzymes, fluorescent materials, radioactive materials, latexes or red blood cells. The labelled anti IgM specific (IgM μ-chain) antibody is caused to react with the solid phase. As a result, an amount of labelled anti human IgM specific antibody, which is proportional to the amount of anti TP IgM antibody as the solid phase, is bonded to the solid phase. Therefore, by measuring quantitatively the labelled material, the amount of anti-TP IgM antibody can be measured.

The total anti-TP antibody present in a test sample may be determined if, in the above-mentioned first reaction, the test sample is caused to react with two solid phase samples at the same time. One of the solid phase samples is subjected to the second reaction as mentioned above, while the other solid phase sample is inter-reacted with labelled anti human IgG antibody. Since it contains the common antigen, the labelled anti IgG is bonded to the solid phase in accordance with the amount of the TP antibody including IgM that has been bonded to the solid phase, and hence the total anti TP antibody bonded to the solid phase can be measured.

Thus, it is possible to use one test sample, to determine simultaneously the amount of the anti TP IgM antibody and the amount of the total anti TP antibody. When the method of this invention is carried out at the initial stages of infection with syphilis, only the anti TP IgM antibody is detected, while the anti TP IgG antibody will be detected after the elapse of time after infection. Both the anti TP IgM antibody and the anti IgG antibody will be detected during an intermediate period.

In a reaction system in which the test sample contains only the anti TP IgM antibody, the anti TP IgM antibody ratio can be obtained if provision is made for obtaining the same result even if any of the labelled anti human IgM antibody and the labelled anti human globulin antibody is employed in the second reaction. In other words, if the quantity of each of the labelled antibodies is adjusted so as to indicate the same absorbance in the colouring reaction. To achieve this result a test sample is subjected to the first reaction and two labelled antibodies are used in the second reaction at the same time as follows:

$$\text{Anti TP IgM antibody ratio (\%)} = \frac{OD_M}{OD_g} \times 100$$

wherein $OD_M$ indicates the absorbance in that case where the labelled anti IgM antibody is employed in the second reaction, while $OD_g$ indicates the absorbance in the case where the labelled anti globulin antibody is employed in the second reaction. Therefore, $OD_g$ corresponds to the amount of total anti TP antibody.

In this way, the ratio of anti TP IgM antibody to anti TP IgG antibody can be obtained and this ratio can be used for the examination of the development of syphilis.

The absolute quantities of the above-mentioned anti TP IgM antibody and the anti TP IgG antibody in the test sample can be obtained. By using IgM or IgG, with known concentration, a standard curve indicating the relationship between the concentration and absorbance, as in a conventional radio immunoassay, and enzyme immunoassay can be obtained. The absolute amount ($\mu g/ml$) of the antibodies can be obtained by use of the standard curve.

The following example illustrates this invention:

## EXAMPLE

TP antigen: Testicles of a rabbit inoculated with TP were extracted under aseptic conditions . TP proliferated in the testicles was isolated by use of 0.075 M solution of sodium citrate and then subjected to ultrasonic processing, centrifugal separation, dialysis and concentration, whereby TP antigen was obtained. The TP antigen was utilised with the following materials:

Solid phase beads: Glass beads were aminated and the TP antigen obtained above was bonded to the glass beads by use of glutaraldehyde as a coupling agent. After masking unused aldehyde groups, solid phase beads were obtained for further use.

Labelled antibodies: Label-1 was prepared by labelling anti human IgM specific (IgM μ-chain) rabbit antibody with peroxidase (enzyme). Label-2 was prepared by labelling anti human IgG rabbit antibody with peroxidase. The labelling of peroxidase were performed in accordance with Nakane's Method.

Antigen-antibody reaction solution: Tris HCl buffer solution (0.1 M) of pH 8.5 containing 2% by weight bovine serum albumin and 0.5 M with respect to sodium chloride was utilised.

Washing solution: Physiological saline solution was utilised as washing solution.

Enzyme reaction solution: Phosphoric acid-citric acid buffer solution (0.1 M, pH = 5.0) containing 2 mM of azino-bis-thiazoline sulphonic acid (ABTS) and 1 mM of $H_2O_2$ was employed as the substrate, and an 0.5% by weight aqueous solution of oxalic acid was employed as the reaction termination solution.

Five test samples (No.1 to No.5) were used, the samples being collected from human sera considered to be infected with syphilis and to be at the initial stages of infection.

For testing each sample, two solid phase beads were placed in a test tube with a diameter of 10 mm. 500 μl of the antigen-antibody reaction solution were added. 10 μl of serum under test were added and the mixture was allowed to stand at $37^{O}C$ for two hours (First reaction). The reaction solution was removed and the solid beads were washed with the washing solution three times. The two solid phase beads were placed in respective test tubes

The antigen-antibody reaction solution prepared above was added to Label-1 and Label-2 to prepare suspensions of Label-1 and Label-2, having the optimum concentration. 500 µl of the suspension of Label-1 were poured into one solid phase bead-containing test tube and 500 µl of the suspension of Label-2 were poured into the other test tube containing the other solid phase bead. After these test tubes had been allowed to stand at 37°C for one hour, reaction solution was removed from each of the test tubes.

Each solid phase bead was washed four times with the washing solution. 1.0 ml of the enzyme reaction solution was then added to each test tube and reaction was allowed to proceed at ambient temperature for 2 hours. 1.0 ml of the reaction termination solution then was added to the reaction mixture. The absorbance of each reaction mixture was measured with light having a wavelength of 420 nm. The absorbance measured by use of a control was subtracted for the absorbance measured by use of Label-1 or Label-2. The amount of anti TP IgM antibody or the amount of the total anti TP antibody corresponded to the absorbance and enabled the anti TP IgM antibody ratio (%) to be calculated. The results are shown in the following table:

TABLE

| Test Sample No. | 1 | 2 | 3 | 4 | 5 |
|---|---|---|---|---|---|
| Amount of anti TP IgM antibody | 0.04 | 0.00 | 0.00 | 0.00 | 0.07 |
| Amount of total anti TP antibody | 0.14 | 0.32 | 0.25 | 0.05 | 0.07 |
| anti TP IgM antibody ratio (%) | 28 % | 0 | 0 | 0 | 100% |

The results show that Sample No.5 was at the initial stage of infection, and that Sample No.1 was infected, but with some time having elapsed after infection. In the cases of Samples No.2, No.3 and No.4, a longer time had passed after infection.

<u>Claims</u>:

1.  A sero-diagnostic method for a disease capable of serodiagnosis, in which method antigen derived from the pathogenic organism of the disease is bonded to a support material to yield organism antigen bonded support material as a solid phase, a test serum sample is caused to react with said solid phase in a first reaction, a labelled anti specific antibody is caused to react with said solid phase in a second reaction to yield a reaction product labelled in an amount corresponding to the amount of anti organism specific antibody present, and the amount of anti organism specific antibody present is measured.

2.  A sero-diagnostic method for a disease capable of serodiagnosis and in which two different antibodies are produced. in the blood at different stages of infection, in which method antigen derived from the pathogenic organism of the disease, is bonded to a plurality of support materials to yield organism antigen bonded support materials as solid phases, a plurality of test serum samples is caused to react simultaneously with said solid phases in a first reaction, said solid phases are equally divided into two groups, a labelled anti first specific antibody is caused to react with each of said solid phases in one group and a labelled anti second specific antibody is caused to react with each of said solid phases in the other group in a  second reaction to yield reaction products labelled in amounts corresponding to the amounts of anti organism first specific antibody or total anti organism antibody and the respective amounts of anti organism first specific antibody and total anti organism antibody in the sample are measured thereby to indicate through the ratio between said amounts, the stage of infection with the disease.

3. A sero-diagnostic method as claimed in claim 1, wherein the disease is syphilis and said antigen is derived from <u>Treponema</u> <u>pallidum</u>, and wherein a labelled anti IgM specific antibody is caused to react with said
solid phase in the second reaction to yield anti TP
IgM antibody in an amount corresponding to the amount of
anti TP IgM antibody present.

4. A sero-diagnostic method as claimed in claim 2,
wherein the disease is syphilis and said antigen is
derived from <u>Treponema</u> <u>pallidum</u>, and wherein a labelled
anti IgM specific antibody is caused to react with each
of said solid phases in one group and a labelled anti
globulin antibody is caused to react with each of said
solid phases in the other group in the second reaction
thereby to yield reaction products labelled in amounts
corresponding to the amounts of anti TP IgM antibody
and total anti TP antibody present in the solid phases
of the respective said groups.

5. A sero-diagnostic method as claimed in any one
of claims 1 to 4, wherein a said solid phase is constituted by beads, a test tube, a tray for hemagglutination
tests, a latex or Sepharose.

6. A sero-diagnostic method as claimed in any one
of the preceding claims, wherein labelling of said antibodies is effected with an enzyme, a fluorescent material,
a radioactive material, a latex or red blood cells.

## DOCUMENTS CONSIDERED TO BE RELEVANT

| Category | Citation of document with indication, where appropriate, of relevant passages | Relevant to claim |
|---|---|---|
| | DE - A - 2 733 380 (BEHRING WERKE)<br><br>* Page 1, claim 1; page 2, claims 7,8; page 3, lines 4-33; page 4, lines 19-32; page 5, lines 4-23; page 7, lines 1-16 *<br>--- | 1,2,5, 6 |
| | GB - A - 1 503 409 (BENT WEEKE)<br><br>* Column 1, lines 9-16; column 2, lines 49-54; column 3, lines 53-60; claims 1 and 10 *<br>--- | 1,2,6 |
| | US - A - 3 650 437 (G.F. SINNINGS)<br><br>* Abstract *<br>--- | |
| | FR - A - 2 126 760 (N.V. ORGANON)<br><br>* Page 3, lines 14-27; page 4, lines 28-35; claims 1,2 *<br>& GB - A - 1 363 565<br>--- | 1,2,5, 6 |
| A | GB - A - 1 185 065 (BAXTER LABS.) | 3,4 |
| A | US - A - 3 600 494 (TOMIZAWA et al.)<br><br>-------- | 3,4 |

### CLASSIFICATION OF THE APPLICATION (Int. Cl.³)

G 01 N 33/54

### TECHNICAL FIELDS SEARCHED (Int. Cl.³)

G 01 N 33/54
G 01 N 33/56
G 01 N 33/58

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | | |
|---|---|---|
| The present search report has been drawn up for all claims | | |
| Place of search<br>The Hague | Date of completion of the search<br>29.06.1981 | Examiner<br>DE LUCA |

EPO Form 1503.1 06.78